Europäisches Patentamt

European Patent Office

Office européen des brevets

(11) Veröffentlichungsnummer: **0 347 927**
**A2**

## (12) EUROPÄISCHE PATENTANMELDUNG

(21) Anmeldenummer: 89111426.6

(22) Anmeldetag: 23.06.89

(51) Int. Cl.⁴: **A61K 31/41**

Die Bezeichnung der Erfindung wurde geändert (Richtlinien für die Prüfung im EPA, A-III, 7.3).

(30) Priorität: 24.06.88 DE 3821392

(43) Veröffentlichungstag der Anmeldung:
27.12.89 Patentblatt 89/52

(84) Benannte Vertragsstaaten:
AT BE CH DE ES FR GB GR IT LI LU NL SE

(71) Anmelder: **A. Nattermann & Cie. GmbH**
**Nattermannallee 1**
**D-5000 Köln 30(DE)**

(72) Erfinder: **Hüther, Andrea Michaela**
**Brauweiler Weg 133**
**D-5000 Köln 41(DE)**
Erfinder: **Parnham, Michael John**
**Aurikelweg 92**
**D-5024 Pulheim(DE)**
Erfinder: **Sauer, Andreas**
**Schillerstrasse 7**
**D-5010 Bergheim(DE)**

(74) Vertreter: **Patentanwaltsbüro Cohausz &**
**Florack et al**
**Postfach 14 01 20 Schumannstrasse 97**
**D-4000 Düsseldorf 1(DE)**

(54) **Verwendung von 2-Phenyl-1,2-benzisoselenazol-3(2H)-on (Ebselen) bei der Behandlung von Malaria.**

(57) Die Erfindung betrifft Arzneimittel zur Therapie von durch Malariaerreger ausgelöste Krankheiten, die 2-Phenyl-1,2-benzisoselenazol-3(2H)-on enthalten, die Verwendung von 2-Phenyl-1,2-benzisoselenazol-3(2H)-on zur Herstellung solcher Arzneimittel bzw. die Verwendung von 2-Phenyl-1,2-benzisoselenazol-3(2H)-on (Ebselen) zur Therapie von durch Malariaerreger bedingte Krankheiten.

EP 0 347 927 A2

## Neue pharmazeutische Verwendung von 2-Phenyl-1,2-benzisoselenazol-3(2H)-on (Ebselen)

Die Erfindung betrifft ein neues Arzneimittel zur Therapie von durch Malaria bedingte Erkrankungen, die 2-Phenyl-1,2-benzisoselenazol-3(2H)-on enthalten, die Verwendung von 2-Phenyl-1,2-benzisoselenazol-3-(2H)-on (Ebselen) zur herstellung von Arzneimitteln zur Behandlung von durch Malariaerreger bedingte Erkrankungen bzw. die Verwendung von 2-Phenyl-1,2-benzisoselenazol-3(2H)-on (Ebselen) zur Therapie van durch Malariaerreger bedingte Erkrankungen.

2-Phenyl-1,2-benzisoselenazol-3(2H)-on ist eine bekannte Substanz, die zur Behandlung etwa von rheumatischen Er rankungen (DE-PS 30 27073, USP 4,352,799) oder von durch oxidativen Streß bedingte Erkrankungen (DE-PS 36 16923) eingesetzt werden kann. Ebselen kann beispielsweise nach dem Verfahren von R. Weber, M Renson, Bulletin de la Soc. Chin. de France 1976 (7/8), S. 1124 - 1126, durch Reaktion von 2-Methylseleno-N-phenyl-benzamid mit Phosphorpentachlorid und anschließender Hydrolyse hergestellt werden.

Malaria (Wechselfieber, Sumpffieber, Paludismus) kommt vorwiegend in Ländern mit tropischem Klima vor, ist praktisch jedoch auf der ganzen Welt zwischen dem 60. nördlichen und dem 40. südlichen Breitengrad anzutreffen. Vor allem in Afrika treten noch zahlreiche Fälle von Malariaerkrankungen auf (1962 - 1988 ständig etwa 200 Millionen Malariakranke mit 1,5 - 2,5 Millionen Todesfällen pro Jahr).

### Malaria

(E.G. Nauck, Handbuch der Tropenkrankheiten, 1967, S. 140 - 186; G. Covell et al., WHO Monograph Series No. 27, 1955; G. Ehrhart, H. Ruschig, Arzneimittel Band V, 2. Auflage 1973, S. 165 - 210 und dort angegebene, weiterführende Literatur. Eine neuere Übersicht zur Chemotherapie der Malaria geben A.K. und M. Saxena in Drug research Vol. 30, p. 222-267 (1986), ed. by E. Jacker.)

Die Erreger der Malaria sind Plasmodien, die durch den Stich der Anopheles-Mücke übertragen werden. Beim Stich der Mücke gelangen Sporozoiten (Sichelkeime) in das Blut des Menschen. Die Sporozoiten wandern über das Gefäßsystem in die Leber und bilden Schizonten, in der sich eine große Anzahl kleinerer Teilungsformen (Merozoiten) entwickeln. Diese befallen im peripheren Blut erstmals die Erythrozyten und entwickeln sich im Blutkörper wieder zu Schizonten und später zu Merozoiten, die dann abermals die roten Bkutkörperchen befallen. Es kommt immer dann zu Fieberanfällen, wenn die roten Blutkörperchen zerfallen und die Merozoiten frei werden. Nach mehreren Merozoiten-Generationen entstehen aus den Merozoiten weibliche (Makrogameten) und männliche (Mikrogametocyten) Geschlechtsformen, die beim Blutsaugen der Anopheles-Mücke in deren Blutkreislauf gelangen und über mehrere Zwischenstufen wieder infektiöse Sporozoiten bilden.

Die klassischen Malariamittel werden nach ihrer Wirkungsart eingeteilt:

a) schizocontide Mittel, die die entsprechenden Formen der Blutphase vernichten,

b) gametocide Mittel, von denen die Geschlechtsformen im Blut des Menschen beeinflußt werden,

c) gewebe-schizocontide Mittel, die die Erreger in der Gewebephase abtöten,

d) sporontocide Mittel, die die geschlechtlichen Generationen in der Mücke beeinflussen.

Generell haben diese Mittel den Nachteil, nur auf eine bestimmte Lebensform der Parasiten einzuwirken, so daß, bedingt durch die bei den Plasmodien im Laufe der Jahre aufgetretenen, z.T. sehr starken Arzneimittelresistenzen, die Präparate oft kombiniert werden müssen. Besonders die sogenannten kreuzresistenten Stämme bestimmter Plasmodienarten tragen erheblich zur Problematik der Malariabekämpfung bei. Zudem sind alle bekannten Malariamittel mehr oder weniger stark mit Nebenwirkungen behaftet, die eine hohe Dosierung oder länger andauernde Verabreichung der Medikamente nicht gestatten.

### Plasmodium berghei, Plasmodium falciparum

In-vitro-Untersuchungen über die Verwendung von Ebselen bei mit Plasmodium berghei infizierten Erythrozyten zeigten die überraschende Fähigkeit des Ebselens, den Einbau und die Aufnahme von Nukleosiden in solche Erythrozyten zu hemmen. Eine Hemmung tritt bereits ab einer Konzentration von 3,16 µmol/l Ebselen auf, wobei eine maximale Hemmung von 80 % nach einer Inkubationszeit von zwei Stunden mit einer Konzentration von 31,6 µmol/l Ebselen erreicht wird. Durch diese Hemmung der Protein- bzw. DNA/RNA-Synthese wird die Lebensdauer und Lebensfähigkeit der infizierten Erythrozyten stark herabgesetzt.

Mit Plasmodium falciparum infizierte Erythrozyten (Parasitengehalt = 2 %) wurden in verschiedenen Entwicklungsstufen (Ringbildung, Throphozoiten- und Schizontenstadium, erneute Ringbildung) mit unterschiedlichen Konzentrationen an Ebselen versetzt. Überraschenderweise wurde nun gefunden, daß das Ebselen in der Lage ist, die Entwicklung dieser Malariaparasiten in allen Entwicklungsstufen zu verhindern, einschließlich des Einfallens der Merozoiten in die Erythrozyten. Das gilt gleichermaßen für Chloroquin-resistente und Chloroquin-nicht-resistente Stämme.

Eine vollständige Verhinderung des Wachstums wird dabei bereits bei einer Konzentration um 40 μmol/l Ebselen innerhalb von 24 Stunden erreicht.

Die Ergebnisse zeigen deutlich die Wirksamkeit des Ebselens bei der Bekämpfung von durch Plasmodien ausgelöste Krankheiten. Zur therapeutischen Verwendung bei Erkrankungen, die durch Malaria-Parasiten ausgelöst werden, wird Ebselen in geeigneter pharmazeutischer Zubereitung, oral oder parenteral in Dosierungen von 10 bis 2000 mg pro Tag, vorzugsweise 30 bis 300 mg pro Tag, in einer Dosis oder mehreren Teildosen, vorzugsweise in 2 bis 3 Teildosen pro Tag, verabreicht.

Die vorliegende Erfindung betrifft ebenfalls pharmazeutische Präparate, welche als Wirkstoff Ebselen enthalten. Bei den erfindungsgemäßen pharmazeutischen Präparaten handelt es sich um solche zur enteralen wie oralen oder reaktalen sowie parenteralen Verabreichung, welche den pharmazeutischen Wirkstoff allein oder zusammen mit einem üblichen, pharmazeutisch anwendbaren Trägermaterial enthalten. Vorteilhafterweise liegt die pharmazeutische Zubereitung des Wirkstoffs in Form von Einzeldosen vor, die auf die gewünschte Verabreichungsform abgestimmt sind, z.B.

Tabletten, Dragees, Kapseln, Suppositorien, Granulate, Lösungen, Emulsionen oder Suspensionen. Die Dosierung der Substanz liegt üblicherweise zwischen 10 und 2000 mg pro Tag, vorzugsweise zwischen 30 und 300 mg pro Tag, und kann in einer Dosis oder mehreren Teildosen, vorzugsweise in 2 bis 3 Teildosen pro Tag, verabreicht werden. Die Herstellung der erfindungsgemäßen Arzneimittel wird durch die folgenden Beispiele näher erläutert:

Beispiel 1

| Tabletten | |
|---|---|
| Ebselen | 30 mg |
| Lactose | 150 mg |
| kristalline Cellulose | 50 mg |
| Calciumcarboxymethylcellulose | 7 mg |
| Magnesiumstearat | 3 mg |

Die aufgeführten Stoffe werden nach üblichem Verfahren gemischt und gepreßt; die Preßlinge können gegebenenfalls mit einem üblichen Filmüberzug versehen werden.

Beispiel 2

| Tabletten | |
|---|---|
| Ebselen | 90 mg |
| kristalline Cellulose | 150 mg |
| Cutina$^R$ HR | 15 mg |
| Hydroxypropylmethylcellulosephthalat | 20 mg |

Herstellung analog Beispiel 1.

Beispiel 3

3

| Kapseln | |
|---|---|
| Ebselen | 30 mg |
| Lactose | 102 mg |
| kristalline Cellulose | 56 mg |
| kolloidales Siliciumdioxid | 2 mg |

Die aufgeführten Stoffe werden nach üblichem Verfahren gemischt, granuliert und in Hartgelatinekapseln abgefüllt.

Beispiel 4

| Kapseln | |
|---|---|
| Ebselen | 50 mg |
| Talkum | 5 mg |
| Aerosil 200 | 10 mg |

Herstellung analog Beispiel 3.

**Ansprüche**

1. Verwendung von 2-Phenyl-1,2-benzisoselenazol-3(2H)-on (Ebselen) zur Herstellung von Arzneimitteln zur Behandlung von durch Malariaerreger bedingte Krankheiten.